# EUROPEAN PATENT SPECIFICATION

(11) **EP 3 824 759 B1**
(45) Date of publication and mention of the grant of the patent: **28.12.2022**
(21) Application number: 19210007.1
(22) Date of filing: 19.11.2019
(51) Int. Cl.: A61L 9/12, A45D 34/02

(54) **DEVICE FOR DIFFUSING VOLATILE SUBSTANCES**
VORRICHTUNG ZUR DIFFUSION FLÜCHTIGER SUBSTANZEN
DISPOSITIF DE DIFFUSION DE SUBSTANCES VOLATILES

(43) Date of publication of application: 26.05.2021
(73) Proprietor: Zobele Holding SpA, 38123 Trento (IT)
(72) Inventor: DEFLORIAN, Stefano, 38123 TRENTO (IT); SORDO, Walter, 38123 TRENTO (IT); GARCIA, Ruben, 08019 BARCELONA (ES)
(74) Representative: Herrero & Asociados, S.L.

(56) References cited:
- EP-A1- 2 962 701
- WO-A1-2017/027759

## Description

The present invention refers to a device for diffusing volatile substances.

### Background of the invention

Among volatile substance diffusers, one of the simplest products existing, and yet still on the top of sales, is impregnated cellulosic support to be hanged for example in a car or in a wardrobe.

Apart the simplest embodiment of cellulose impregnated at the factory, some improvements have been proposed where a container containing the liquid is perforated to release the liquid in a porous element from which it is then released in the atmosphere.

Examples of this kind of volatile substance diffusers are disclosed in US4526320, US4762275, US4161284, DE2807424, GB2228681, and GB2349089.

EP2962701A1 discloses an easy-to-break carrying device for a volatile including a packaging case, a sealing film, a destruction assembly, and a permeable film. The packaging case has a first accommodating space for receiving the volatile and a second accommodating space connecting to the first accommodating space having an opening.

WO2017027759A1 discloses a volatile composition dispenser comprising a reservoir containing a volatile composition and sealed by a substrate, a membrane surrounding at least part of the sealed reservoir, and a rupture mechanism formed of a single piece of plastic located between the membrane and the sealed reservoir.

Most of these diffusers comprise an activation part including a wide sharp tip or needle that is placed in proximity of a breakable portion of the container.

From these documents, it is clear from a skilled person that it is very challenging to have such a sharp tip in contact with a breakable foil and not to have an accidental activation of the system, either during the assembly process or during transportation.

Another drawback of the current diffusers is that the needle or sharp tip is big, forming a big hole in the breakable portion of the container, that could create non-wanted alteration of the aesthetics of the refill, especially if this part is used for containing the brand of the product.

### Description of the invention

Therefore, one purpose of the present invention is to provide a device for diffusing volatile substances where an accidental leakage is avoided.

With the device for diffusing volatile substances according to the present invention it is possible to solve said drawbacks, providing other advantages that are described below.

The device for diffusing volatile substances according to the present invention is defined in claim 1.

Furthermore, said at least one perforating element is preferably mounted on a plate, which when the perforable sheet is perforated, is in contact with the porous element.

According to a preferred embodiment, the weakened area is a cut or several cuts made in said porous element, e.g. the weakened are is formed by two crossing cuts defining a central point.

Advantageously, said plate includes two or more perforating elements, wherein the perforating elements in said plate are placed vertically, defining an upper perforating element and a lower perforating element.

According to this embodiment, the container is partially filled with the volatile substance, defining a filled portion and an empty portion in the container so that the upper perforating element is at a height corresponding to the empty portion and the lower perforating element is at a height corresponding to the filled portion.

To prevent the removal of the perforating element(s), the or each perforating element comprises a stopper at its tip. The stopper prevents the activation system to go back and the top side can show that the perforation has happen and it does not have to be repeated by the user.

At the same time, if the distance between the stopper and the top part is the same thickness of the porous element, the top part will remain in contact with the porous element and will prevent the likelihood of liquid to freely come out from the hole created between the perforable sheet and the external surface of porous element.

With the device according to the present invention, the perforation of the perforable sheet can be made easily, and once perforated, an accidental leakage is prevented.

### Brief description of the drawings

For a better understanding the above explanation and for the sole purpose of providing an example, some non-limiting drawings are included that schematically depict a practical embodiment.

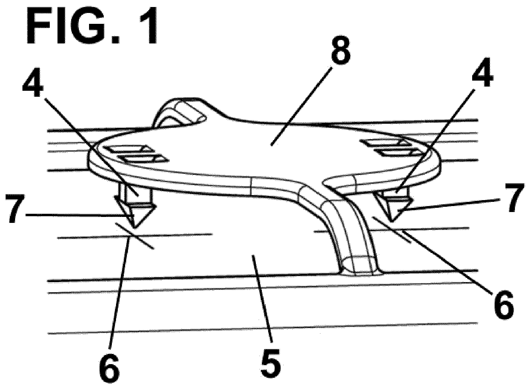

Figure 1 is a perspective of a portion of the device for diffusing volatile substances according to the present invention;
Figure 2 is a sectional view of a portion of the device for diffusing volatile substances according to the present invention before its activation; and
Figure 3 is a sectional view of a portion of the device for diffusing volatile substances according to the present invention after its activation, when the perforation element has perforated the perforable sheet.

### Description of a preferred embodiment

The present invention refers to a device for diffusing volatile substances, such as perfume or insecticide, comprising a container 1 containing a liquid volatile substance 2, having at least on part of it that is easily perforable sheet 3.

It must be pointed out that the device according to the present invention can comprise more that one container, but for simplicity reasons only one container is shown in the drawings.

The device for diffusing volatile substances according to the present invention also comprises one or more perforating elements 4, such as needles, which are designed to be able to perforate the perforable sheet 3 of the container 1 when user press them against the perforable sheet 3.

The device for diffusing volatile substances according to the present invention also comprises a porous element 5 that is able to absorb and contain the liquid after the perforable sheet 3 of the container 1 has been perforated.

This porous element 5 is placed between the perforating elements 4 and the perforable sheet 3, and the porous element 5 comprises a weakened area 6 in the proximity of the perforating elements 4.

Furthermore, the perforating elements 4 have a design that avoid that it goes back after perforating the perforable sheet 3 of the container 1. To this end, e.g. each perforating element 4 has a stopper 7 or a portion with greater dimensions than the rest of the perforating element 4, e.g. an arrow shape, i.e. the cross section of the perforation element 4 is progressively wider distally from its tip.

This way, after perforating the perforable sheet 3, the perforating element 4 cannot be removed from the container 1, avoiding an accidental leakage.

According to the embodiment shown in the drawing, two perforating elements 4 are mounted on a plate 8 that covers the external part of the weakened area of the porous element 5.

As the perforating elements 4 are not able to be removed from the container 1, the plate 8 is maintained in physical contact with the porous element 5 after activation, avoiding liquid to flow freely through the weakened area 6 of the porous element 5.

Preferably, the container 1 is made from a plastic cup, thermoformed, closed by a rupturing foil.

The rupturing foil is preferably a very thin aluminium foil with a sealing layer on one side and a protective lacquer on the other.

The porous element 5 is preferably a cellulosic support. Alternatively, it could be made of plastic fibers or plastic foam.

The weakened area 6, according to a preferred embodiment, is a cut or a partial cut with a cross shape, where the center of the cross matches with the tip of the perforating element 4.

Alternatively, the weakened area 6 can be made by a hole in the porous element 5.

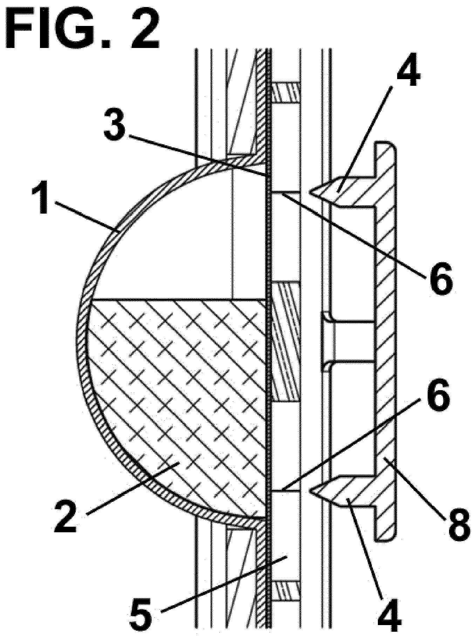

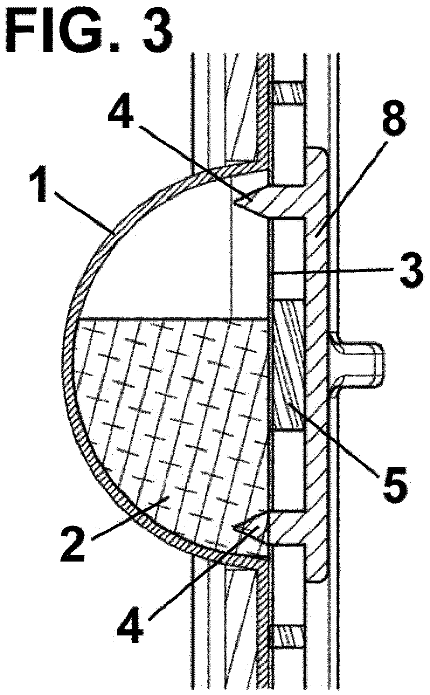

According to an embodiment, shown in Figs. 2 and 3, the device according to the present invention comprises two perforating elements 4, placed vertically to each other, where the upper perforating element 4 is at a height where, inside the container 1, there is no liquid, and the lower perforating element 4 is at a height corresponding to the bottom of the container 1. This way, the user can form a hole in the perforable sheet 3 yet still having the liquid to flow through it (the upper hole aim is to allow air to enter in the container 1).

In a preferred embodiment, the device according to the present invention is a device to be hanged to a car mirror with an overall dimension of e.g. 13 × 8 cm.

The device can comprise a U-shape plastic frame, preferably made of polypropylene, where four containers 1 can be made, containing the liquid volatile substance 2.

The perimetric wall of the plastic frame is done with a U-shape profile in order to guide and retain the porous element 5 in position.

In the back part of the frame, four plates 8 with activation perforating elements 4 can be formed, each plate for one container. These plates 8 can be individually pressed by a user to force the perforating elements 4 to perforate the perforable sheet 3.

The four containers 1 are preferably filled with the same amount and kind of fragrance. E.g. each container contains the volume of fragrance equivalent of a duration of 1 week.

For using the device according to the present invention, the user just activates it pressing on the plate 8 so that the perforating element(s) 4 perforate(s) the perforable 3, and the porous element 5 is impregnated with the liquid volatile substance 2.

Even though reference has been made to a specific embodiment of the invention, it is obvious for a person skilled in the art that the device for diffusing volatile substances described herein is susceptible to numerous variations and modifications, and that all of the details mentioned can be substituted for other technically equivalent ones without departing from the scope of protection defined by the attached claims.

## Claims

1. Device for diffusing volatile substances, comprising:
- a container (1) that contains a volatile substance (2);
- a perforable sheet (3) that closes at least a portion of said container (1);
- at least one perforating element (4)
**characterized in that** the device comprises:
- a porous element (5) placed between said perforable sheet (3) and said perforating element (4), comprising at least one weakened area (6) in correspondence with said at least one perforating element (4), said perforating element (4) is configured to perforate the perforable sheet (3) and the porous element (5) simultaneously, said porous element (5) being impregnated with the volatile substance (2) when the perforable sheet (3) is perforated.

2. Device for diffusing volatile substances according to claim 1, wherein said at least one perforating element (4) is mounted on a plate (8), which when the perforable sheet (3) is perforated, is in contact with the porous element (5).

3. Device for diffusing volatile substances according to claim 1, wherein the weakened area (6) is a cut or several cuts made in said porous element (5).

4. Device for diffusing volatile substances according to claim 3, wherein the weakened area (6) is formed by two crossing cuts defining a central point.

5. Device for diffusing volatile substances according to claim 2, wherein said plate (8) includes two or more perforating elements (4).

6. Device for diffusing volatile substances according to claim 1, wherein the or each perforating element (4) comprises a stopper (7) at its tip.

7. Device for diffusing volatile substances according to claim 5, wherein the perforating elements (4) in said plate are placed vertically, defining an upper perforating element and a lower perforating element.

8. Device for diffusing volatile substances according to claim 7, wherein the container (1) is partially filled with the volatile substance (2), defining a filled portion and an empty portion in the container (1) so that the upper perforating element is at a height corresponding to the empty portion and the lower perforating element is at a height corresponding to the filled portion.

## Patentansprüche

1. Vorrichtung zur Diffusion von flüchtigen Substanzen, die umfasst:
- einen Behälter (1), der eine flüchtige Substanz (2) enthält;
- eine perforierbare Folie (3), die wenigstens einen Teil des Behälters (1) verschließt:
- wenigstens ein Perforationselement (4),
**dadurch gekennzeichnet, dass** die Vorrichtung umfasst:
- ein zwischen der perforierbaren Folie (3) und dem Perforationselement (4) platziertes poröses Element (5), das wenigstens einen geschwächten Bereich (6) entsprechend dem wenigstens einen Perforationselement (4) umfasst, wobei das Perforationselement (4) konfiguriert ist, um die perforierbare Folie (3) und das poröse Element (5) gleichzeitig zu perforieren, wobei das poröse Element (5) mit der flüchtigen Substanz (2) imprägniert wird, wenn die perforierbare Folie (3) perforiert wird.

2. Vorrichtung zur Diffusion flüchtiger Substanzen nach Anspruch 1, wobei das wenigstens eine Perforationselement (4) auf einer Platte (8) montiert ist, die, wenn die perforierbare Folie (3) perforiert wird, mit dem porösen Element (5) in Kontakt ist.

3. Vorrichtung zur Diffusion flüchtiger Substanzen nach Anspruch 1, wobei der geschwächte Bereich (6) ein Schnitt oder mehrere Schnitte sind, die in dem porösen Element (5) angefertigt werden.

4. Vorrichtung zur Diffusion flüchtiger Substanzen nach Anspruch 3, wobei der geschwächte Bereich (6) durch zwei sich kreuzende Schnitte gebildet wird, die einen zentralen Punkt definieren.

5. Vorrichtung zur Diffusion flüchtiger Substanzen nach Anspruch 2, wobei die Platte (8) zwei oder mehrere Perforationselemente (4) aufweist.

6. Vorrichtung zur Diffusion flüchtiger Substanzen nach Anspruch 1, wobei das oder jedes Perforationselement (4) an seiner Spitze einen Stopfen (7) umfasst.

7. Vorrichtung zur Diffusion flüchtiger Substanzen nach Anspruch 5, wobei die Perforationselemente (4) in der Platte vertikal platziert sind, wobei sie ein oberes Perforationselement und ein unteres Perforationselement definieren.

8. Vorrichtung zur Diffusion flüchtiger Substanzen nach Anspruch 7, wobei der Behälter (1) mit der flüchtigen Substanz (2) teilweise gefüllt ist, wobei ein gefüllter und ein leerer Teil im Behälter (1) definiert wird, so dass sich das obere Perforationselement an einer Höhe entsprechend dem leeren Teil und das untere Perforationselement an einer Höhe entsprechend dem gefüllten Teil befindet.

## Revendications

1. Dispositif de diffusion de substances volatiles, comprenant :
- un contenant (1) qui contient une substance volatile (2);
- une feuille perforable (3) qui ferme au moins une partie dudit conteneur (1) ;
- au moins un élément perforateur (4)
**caractérisé en ce que** le dispositif comprend :
- un élément poreux (5) placé entre ladite feuille perforable (3) et ledit élément perforateur (4), comprenant au moins une zone affaiblie (6) en correspondance avec ledit au moins un élément perforateur (4), ledit élément perforateur (4) étant configuré pour perforer la feuille perforable (3) et l'élément poreux (5) simultanément, ledit élément poreux (5) étant imprégné de la substance volatile (2) lorsque la feuille perforable (3) est perforée.

2. Dispositif de diffusion de substances volatiles selon la revendication 1, dans lequel ledit au moins un élément perforateur (4) est monté sur une plaque (8), qui lorsque la feuille perforable (3) est perforée, est en contact avec l'élément poreux (5).

3. Dispositif de diffusion de substances volatiles selon la revendication 1, dans lequel la zone affaiblie (6) est une entaille ou plusieurs entailles effectuées dans ledit élément poreux (5).

4. Dispositif de diffusion de substances volatiles selon la revendication 3, dans lequel la zone affaiblie (6) est formée par deux entailles entrecroisées définissant un point central.

5. Dispositif de diffusion de substances volatiles selon la revendication 2, dans lequel ladite plaque (8) comprend deux ou plusieurs éléments perforateurs (4).

6. Dispositif de diffusion de substances volatiles selon la revendication 1, dans lequel le ou chaque élément perforateur (4) comprend un bouchon (7) à son extrémité.

7. Dispositif de diffusion de substances volatiles selon la revendication 5, dans lequel les éléments perforateurs (4) de ladite plaque sont placés verticalement, définissant un élément perforateur supérieur et un élément perforateur inférieur.

8. Dispositif de diffusion de substances volatiles selon la revendication 7, dans lequel le contenant (1) est partiellement rempli de la substance volatile (2), définissant une partie remplie et une partie vide dans le contenant (1) de sorte que l'élément perforateur supérieur soit à une hauteur correspondant à la partie vide et que l'élément perforateur inférieur soit à une hauteur correspondant à la partie remplie.
